# EUROPEAN PATENT APPLICATION

(11) **EP 2 063 611 A1**
(43) Date of publication of application: **27.05.2009**
(21) Application number: 07121104.9
(22) Date of filing: 20.11.2007
(51) Int. Cl.: H04M 1/247, G09B 21/00, A61F 11/04

(54) **Mobile communication device as assistive device for hearing impaired people**

(71) Applicant: LUCENT TECHNOLOGIES INC., Murray Hill NJ 07974-0636 (US)
(72) Inventor: Willigenburg, Willem, 1223 JR, Hilversum (NL)
(74) Representative: MERH-IP Matias Erny Reichl Hoffmann

(57) **Abstract**

This invention relates to a method and an apparatus for adapting a mobile communication device to the benefit of hearing impaired people. Mobile communication devices today comprise microphones, which can be used to detect sound events and vibrating devices, which can be used to generate vibration events. A service logic, which correlates predefined sound patterns with predefined vibration patterns, is proposed in order to help hearing impaired people to detect sound events, which they cannot hear, via a vibration event generated by the vibrating device of the mobile communication device.

## Description

This invention relates to a method and an apparatus for adapting a mobile communication device to the benefit of hearing impaired people.

A hearing impairment or hearing loss is a full or partial decrease in the ability to detect or understand sounds. It can be caused by a wide range of biological and environmental factors. Loss of the ability to detect some frequencies, or to detect low-amplitude sounds, that a healthy person naturally detects, is a hearing impairment. The complete lack of ability to detect sound is generally referred to as deafness. The global deaf population is roughly estimated to be 0.1 % of the total population (i.e. 1 in 1000).

Many hearing impaired individuals use assistive devices in their daily lives. Individuals can communicate by telephone using a telecommunication device for the deaf (TDD). This device looks like a typewriter or word processor and transmits typed text over the telephone. Since recently, mobile TDD devices exist allowing simultaneous two-way text communication. In the U.S. and many other western countries there are telephone relay services so that a hearing impaired person can communicate with a hearing person via a human translator. Internet and mobile phone/SMS text messaging are beginning to take over the role of the TDD. Video conferencing is also a new technology that permits signed conversations as well as permitting a sign language interpreter to voice and sign conversations between a hearing impaired and a hearing person.

With SMS text messaging services becoming more and more popular among the group of hearing impaired people, some communication possibilities via standard mobile communication devices are becoming largely available. These mobile communication devices comprise a certain number of components, such as a microphone and in many cases a vibrating device, used to supplement the ring tone in order to signal an incoming call or an incoming SMS.

According to an aspect of the present invention, a mobile communication device is used to detect a sound event via its microphone and to generate a related vibration event using its vibrating device.

According to another aspect of the invention, the mobile communication device comprises a service logic which controls the generation of a vibration event in response to the sound event detected by the microphone of the mobile communication device.

According to another aspect of the invention, the service logic is configured such that end-users can select from a certain number of modes, such as - inter alia - a dance mode, a movie mode, a traffic mode and a childcare mode. The selection of the modes may be assisted by a menu. The modes may be fine-tunable by the end-user. For instance, parameters of the vibrating device such as the strength, the duration and the rhythm of the vibration event may be selectable. In addition, parameters of the microphone, such as its sensitivity, may be adjustable. Furthermore, the parameters of the service logic, such as threshold levels, sound patterns, vibration patterns, etc., may be fine-tunable.

For clarity it is to be understood that a sound event is detected via the microphone of the mobile communication device and represented by a sound pattern. The mobile communication devise may comprise a plurality of sound patterns stored in a memory of the mobile communication device. Such sound patterns may include user-independent patterns, such as the typical sound of a honking horn of a car, and user-dependent patterns, such as a spoken word of a specific person. Using pattern matching techniques known to a person skilled in the art, the sound event is compared to the sound patterns stored in the memory of the mobile communication device and the best matching pattern is selected. Parameters and thresholds may be modifiable to fine-tune the performance of the matching techniques and to determine if a matching sound pattern was recognized or not.

On the other side, a vibration event is generated by the vibrating device according to a vibration pattern defined in the mobile communication device. Such vibration patterns may be stored in a memory of the mobile communication device and define a set of parameters that will control the modulation over time of the vibrating device. This way, a predefined vibration event is generated.

According to another aspect of the invention, the end-user may add additional personal profiles and modes of his choice to the service logic of the mobile communication device, which relate certain sound events to vibration events.

According to another aspect of this invention, the service logic could be purely based on software, which can be loaded onto standard mobile communication devices. This would allow hearing impaired people to upgrade their existing mobile communication devices to an assistive device according to the present invention. Such upgrades are possible today, as mobile communication devices have sufficient processor and memory characteristics and as the operating systems running on the mobile communication devices provide application programming interfaces (APIs), which allow the development and the execution of such software applications.

Certain modes, however, might require additional or modified hardware. By way of example, the vibrating device of some conventional mobile communication devices might not yield the desired performance, when representing the rhythm of a music sound event. It could also be that the processing capabilities of some conventional mobile communication devices might not be able to execute the service logic in the short time frames required by a particular mode. In such cases, it might be beneficial to design mobile communication device hardware specifically tailored to the present invention. Such mobile communication devices may comprise special purpose microphones that may have specific directivity and/or frequency response characteristics. The vibrating device might be specifically designed to allow for a large variety of vibration events. Furthermore, the processor performance and the working memory characteristics may be altered, in order to cope with the real-time requirements of a sound matching technique.

The features and advantages of the invention will become apparent from the following description of preferred embodiments. The present invention is described in the following by referring to exemplary embodiments illustrated schematically in the accompanying figure, wherein

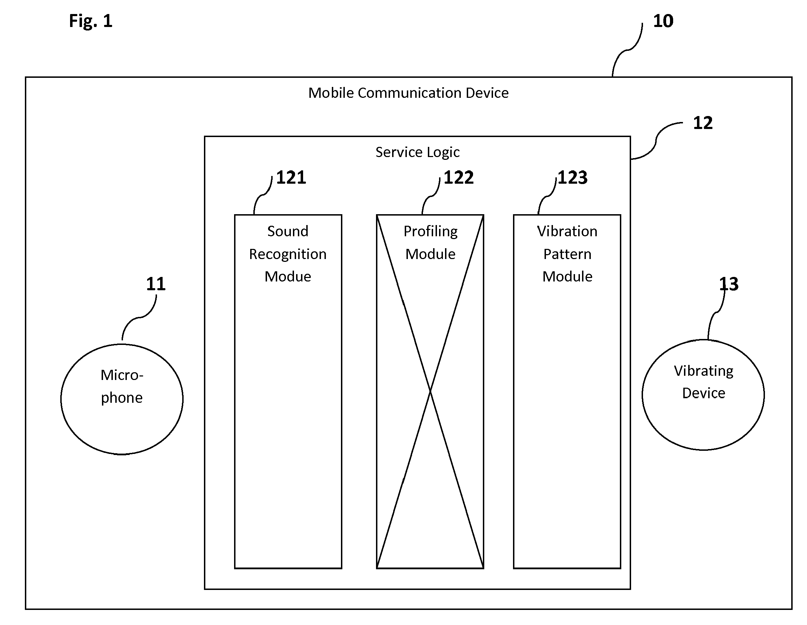

Fig. 1 illustrates a mobile communication device 10 comprising a service logic as disclosed in the present invention.

The mobile communication device 10 comprises a microphone 11 and a vibrating device 13. Preferably, the microphone 11 is adapted to support a hands-free mode of the mobile communication device 10, therefore enabling it to detect sounds that are in the greater surrounds at a longer distance of the mobile communication device 10. The vibrating device 13 is normally implemented by a small electric motor connected to an eccentric (unbalanced) weight.

The mobile communication device 10 also comprises a service logic 12. The service logic 12 comprises a sound recognition module 121, a vibration pattern module 123 and a profiling module 122.

The sound recognition module 121 enables the device to detect specific sound patterns (e.g. the sound of a base drum or the sound of a honking horn) and/or specific voice patterns (e.g. the cry of a specific baby). The sound recognition module 121 may provide multiple parameters to fine tune its performance. Possible embodiments of such sound recognition modules 121 and its tuning parameters are readily known to a person skilled in the art.

The vibration pattern module 123 allows the end-user to define different vibration patterns, a vibration pattern being defined by a plurality of parameters, such as strength, duration, rhythm, increasing or decreasing amplitudes, etc. Using the vibration pattern module 123, the end-user can compose complete vibration patterns and store them for later use.

In the profiling module 122, the end-user can correlate the sound patterns specified in the sound recognition module 121 with the vibration patterns specified in the vibration pattern module 123 to form usage profiles or modes, such as a dance mode, a movie mode, a traffic mode or a childcare mode.

In an embodiment of the invention, a dance mode will capture the rhythm of a base drum via the sound recognition module 121. Each hit of the base drum will be immediately translated by the profiling module 122 into a vibration pattern specified in the vibration pattern module 123. For a dance application it might be beneficial to set a relatively short and strong vibration pattern.

In another embodiment of the invention, a traffic mode will capture multiple traffic sounds, such as a honking horn or an approaching car via the sound recognition module 121. The honking horn will be translated immediately by the profiling module 122 into a short and strong vibration pattern. The approaching car, on the other hand will be translated by the profiling module 122 into a vibration pattern with an amplitude that increases in parallel to the approaching car. As can be seen from this example, different sound events can be correlated by the profiling module 122 with different vibration patterns. This will enable the hearing impaired person to distinguish between multiple sound events.

In another embodiment of the invention, a childcare mode will capture the cry of a specific baby and/or the word "mum", spoken by a specific child, via the voice recognition module 121. Voice recognition means may be applied to ensure that only the voices of predefined children and/or adults are captured. The profiling module 122 translates these sound events into vibration patterns, assigning for example a short single vibration impulse to the cry of a baby and the spoken word "mum" to a double vibration impulse. This way, the hearing impaired person will be able to distinguish between the crying baby and the child that seeks attention.

Usually, a mobile communication device 10 will be provided with preset profiles and modes, however, in an embodiment of the invention, it will be possible to set user-defined profiles and modes. In these, the end-user can correlate self-defined or pre-defined sound patterns with self-defined or pre-defined vibration patterns.

Using the present invention, an assistive device for hearing impaired people can be provided on the basis of a mobile communication device. Such an assistive device could significantly improve the social life of hearing impaired people. On the other side, a value-added functionality for mobile communication devices is proposed, which could improve the sale of such mobile communication devices. As in some embodiments of the invention, the proposed service logic does not require any hardware modifications, the value of a mobile communication device could be improved without increasing the manufacturing costs of the device itself.

The present invention is not limited to the disclosed exemplary applications. Other applications can benefit from the invention as well. This written description uses examples to disclose the invention, including the best mode, and also to enable any person skilled in the art to make and use the invention. While the invention has been described in terms of various specific embodiments, those skilled in the art will recognise that the invention can be practiced with modification within the spirit and scope of the claims. Especially, mutually non-exclusive features of the embodiments described above may be combined with each other. The patentable scope of the invention is defined by the claims, and may include other examples that occur to those skilled in the art.

## Claims

1. A mobile communication device (10) comprising a microphone (11) and a vibrating device (13), whereas the mobile communication device (10) is configured to recognize sound events via the microphone (11) and to generate vibration events using the vibrating device (13), whereas the vibration events are correlated with the sound events.

2. The device according to claim 1, wherein the device comprises a service logic (12) allowing a user to select and to configure a plurality of modes which correlate a plurality of sound patterns, representing sound events captured via the microphone (11), to a plurality of vibration patterns, representing vibration events to be generated by the vibrating device (13).

3. The device according to claim 2, wherein correlations between sound patterns and vibration patterns are adaptable to the preferences of the end-user by setting parameters of the microphone (11) and parameters of the vibrating device (13) within the service logic (12).

4. The device according to claim 3, wherein the service logic (12) comprises a traffic mode, which creates vibration events representing sound events captured from the traffic, such sound events comprising - inter alia - a pick up horn or the sound of a passing car.

5. The device according to claim 3, wherein the service logic (12) comprises a childcare mode, which creates vibration events representing sound events captured from children, such sound events comprising - inter alia - the cry of a baby.

6. The device according to claim 3, wherein the service logic (12) comprises a recognition unit for detecting sound events.

7. The device according to claim 6, wherein the recognition unit comprises a speech recognition unit for detecting sound events that relate to words spoken and/or sounds created by a specific person.

8. The device according to claim 3, wherein the device is adapted for translating sound events into vibration events for the benefit of hearing impaired people.

9. A method for usage in a mobile communication device, **characterized in that** vibration events are generated using a vibrating device of the mobile communication device in response to sound events captured via a microphone of the mobile communication device.

10. A method according to claim 9, wherein software is uploaded on a standard mobile communication device, and wherein the software implements a service logic which correlates the said vibration events with the said sound events.
